# NEW EUROPEAN PATENT SPECIFICATION

(11) **EP 0 446 869 B2**
(45) Date of publication and mention of the opposition decision: **12.02.2003**
(45) Mention of the grant of the patent: 20.03.1996
(21) Application number: 91103753.9
(22) Date of filing: 12.03.1991
(51) Int. Cl.: C07C 19/08, C07C 17/20, C07C 17/08

(54) **Process for preparing 1,1,1-trifluorochloroethane and 1,1,1,2-tetrafluoroethane**
Verfahren zur Herstellung von 1,1,1-Trifluorchlorethan und 1,1,1,2-Tetrafluorethan
Procédé pour la fabrication d'1,1,1-trifluorochloroéthane et d'1,1,1,2-tétrafluoroéthane

(30) Priority: 13.03.1990 JP 6181190; 22.10.1990 JP 28559690
(43) Date of publication of application: 18.09.1991
(60) Divisional application: 94105545.1
(73) Proprietor: DAIKIN INDUSTRIES, LIMITED, Osaka-shi, Osaka-fu 530 (JP)
(72) Inventor: Koyama, Satoshi, c/o Yodogawaseisakusho of, Settsu-shi, Osaka-fu (JP); Homoto, Yukio, c/o Yodogawaseisakusho of, Settsu-shi, Osaka-fu (JP); Esaka, Naoki, c/o Yodogawaseisakusho of, Settsu-shi, Osaka-fu (JP)
(74) Representative: Hansen, Bernd, Dr. Dipl.-Chem.

(56) References cited:
- EP-A- 0 295 885
- EP-A- 0 328 127
- EP-A- 0 366 797
- EP-A- 0 408 005
- WO-A-90/08755
- GB-A- 1 000 485
- GB-A- 1 589 924
- GB-A- 2 030 981
- JP-A- 4 872 105
- US-A- 3 752 850
- US-A- 4 129 603
- US-A- 4 792 643
- G. Natta et al, "Principi della Chimica Industriale", p. 510-511

## Description

The present invention relates to a process for preparing 1,1,1,2-tetrafluoroethane. More particularly, the present invention relates to a process for preparing 1,1,1-trifluorochloroethane by reacting trichloroethylene and hydrogen fluoride and preparing 1,1,1,2-tetrafluoroethane by further fluorinating 1,1,1-trifluorochloroethane.

### Description of the Related Art

1,1,1,2-Tetrafluoroethane (hereinafter referred to as "R-134a") is a promising substitute for dichlorodifluoromethane (R-12) which is widely used as a refrigerant, and it is highly desired to establish a process for producing R-134a. 1,1,1-Trifluorochloroethane (hereinafter referred to as "R-133a") is useful as an intermediate in the preparation of R-134a or a raw material for the preparation of trifluoroethanol.

Various processes are known for the preparation of R-134a but each process has its own advantages and disadvantages.

For example, in a process comprising reducing CF₃CCl₂F (R-114a) with hydrogen, a conversion is high but a life of a catalyst is very short.

In a process comprising reacting trichloroethylene and hydrogen fluoride to obtain R-133a and then fluorinating R-133a with hydrogen fluoride in a gas phase (cf. Japanese Patent Kokai Publication No. 72105/1973), a selectivity is high and a life of a catalyst is long, but the process has the following drawbacks:
1. Since the reaction for fluorinating trichloroethylene is an exothermic reaction which generates a large amount of heat (about 30 Kcal./mole), control of the reaction is difficult.
2. Since, in the fluorination step of R-133a, 1,1-difluorochloroethylene (hereinafter referred to as "R-1122) which forms an azeotropic mixture with R-134a is contained in the reaction mixture, it is difficult to separate R-134a from the reaction mixture.

WO-A-90 08755 and EP-A-0 408 005 have publication dates which fall after the earliest priority date of the present application. WO-A-90 08755 provides a process for the manufacture of 1,1,1,2-tetrafluoroethane by reacting HF and trichloroethylene in the presence of a catalyst to form a mixture comprising 2-chloro-1,1,1-trifluoroethane and 1,1,1,2-tetrafluoroethane. The reaction is conducted in a single reaction zone after which the 1,1,1,2-tetrafluoroethane is recovered from the mixture whereas the 2,chloro-1,1,1-trifluoroethane and other organic by-products together with trichloroethylene and HF are recycled.

EP-A-0 408 005 discloses a process for preparing 1,1,1,2-tetrafluoroethane by reacting trichloroethylene, 1,1,1-trifluorochloroethane and hydrofluoric acid in the gas phase in the presence of a chromium trioxide catalyst The process can be made continuous by recycling unreacted trichloroethylene and 1,1,1-trifluorochloroethane in order to compensate for the relatively low total conversion of the starting materials.

EP-A-0 295 885 discloses the production of a chromium oxide based catalyst for use in the fluorination of inter alia trichloroethane to form 1,1,1,2-tetrafluoroethane. It is noted that the compound 1,1,1-trifluoro-2-chloroethane acts as an intermediate and that any of this compound present in the final product may be recycled.

### SUMMARY OF THE INVENTION

One object of the present invention is to provide a process for preparing 1,1,1,2-tetrafluoroethane with a simple apparatus at a reduced cost.

According to a first aspect of the present invention, there is provided a process for preparing 1,1,1,2-tetrafluoroethane comprising the steps of:
(i) fluorinating trichloroethylene in the gas phase with hydrogen fluoride in the presence of a fluorination catalyst in a first reactor to form 1,1,1-trifluoro-2-chloroethane; and
(ii) fluorinating the 1,1,1-trifluoro-2-chloroethane in the gas phase with hydrogen fluoride in the presence of a fluorination catalyst in a second reactor to generate a first gaseous mixture comprising 1,1,1,2-tetrafluoroethane, unreacted 1,1,1-trifluoro-2-chloroethane and 1,1-difluorochloroethylene as a by-product
characterized in that:
(a) at least a part of the first gaseous mixture is fed to the first reactor where it acts as a diluent for the fluorination step (i) and where the 1,1-difluorochloroethylene is fluorinated with hydrogen fluoride to 1,1,1-trifluoro-2-chloroethane; and
(b) the 1,1,1,2-tetrafluoroethane is recovered from the mixture of gases resulting from the fluorination step (i).

According to a second aspect, the present invention provides a process for preparing 1,1,1,2-tetrafluoroethane comprising the steps of:
(1) reacting trichloroethylene with hydrogen fluoride in the gas phase in the presence of a fluorination catalyst to obtain 1,1,1-trifluoro-2-chloroethane in a first reactor,
(2) reacting 1,1,1-trifluoro-2-chloroethane from the first reactor with hydrogen fluoride in the gas phase in the presence of a fluorination catalyst to obtain 1,1,1,2-tetrafluoroethane and by-product 1,1-difluorochloroethylene in a second reactor,
(3) recycling the entire reaction mixture including 1,1,1,2-tetrafluoroethane and unreacted 1,1,1-trifluoro-2-chloroethane from the second reactor to the first reactor,
(4) reacting 1,1-difluorochloroethylene produced in the second reactor with hydrogen fluoride to reduce the amount of 1,1,-difluorochloroethylene in the first reactor, and,
(5) recovering 1,1,1,2-tetrafluoroethane from the reaction mixture obtained from the first reactor prior to feeding this mixture to the second reactor.

The present invention has been completed based on the finding that, when trichloroethylene and hydrogen fluoride are diluted with the diluent gas which is inactive to the reaction, it is very easy to control their reaction temperature, and when a generated gas from the reaction of R-133a and hydrogen fluoride is used as the diluent gas, generation of R-1122 is suppressed to a very low level while not influencing the reaction between trichloroethylene and hydrogen fluoride.

### BRIEF DESCRIPTION OF THE DRAWING

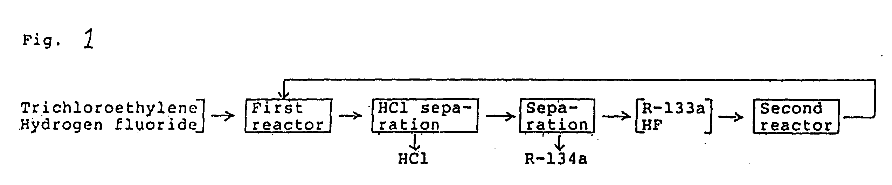

Fig. 1 is a flow chart of of a process according to one embodiment of the present invention.

### DETAILED DESCRIPTION OF THE INVENTION

The process of the present invention will now be explained in detail.

To carry out the first reaction, trichloroethylene and hydrogen fluoride are supplied to a first reactor containing the diluent gas derived from the second reactor. When the diluent gas contains a sufficient amount of hydrogen fluoride, it is not necessary to supply hydrogen fluoride. The amount of hydrogen fluoride is from 3 to 100 moles per one mole of trichloroethylene. When the amount of hydrogen fluoride is smaller than the lower limit, an amount of unreacted trichloroethylene increases though the reaction may proceed. When the amount of hydrogen fluoride is larger than the upper limit, the reactor becomes large and the process becomes uneconomical.

An amount of the diluent gas is not critical. While the amount of the diluent gas has some influence on the control of reaction temperature, the reaction temperature can be controlled more or less. However, when a very large amount of the diluent gas is used, the reactor becomes large. Accordingly, the volume of the diluent gas is usually from 1 to 40 times the volume of trichloroethylene.

The reaction temperature is preferably from 180 to 300°C. When the reaction temperature is higher than 300°C, R-134a reacts with hydrogen chloride which is generated from the reaction between trichloroethylene and hydrogen fluoride and is reconverted to R-133a

The diluent gas fed to the first reactor from the second reactor may contain up to about 25 % by mole of R-1122. R-1122 is converted to R-133a in the presence of hydrogen fluoride. When an azeotropic mixture of R-1122 with R-134a is used as the diluent gas, R-1122 is converted to R-133a whereby the amount of R-1122 is decreased. In this case, the reaction temperature is from 180 to 300°C for the effective decrease of R-1122. When the reaction temperature is lower than 180°C, the reaction rate of trichloroethylene with hydrogen fluoride is small, and when the reaction temperature is higher than 300°C, R-1122 remains unconverted.

In the process of the present invention, fluorination catalysts are used. As the catalyst, any one that has a catalytic activity on the fluorination reaction can be used. In general, chromium oxide base catalysts are used. Examples are thermally treated Cr(OH)₃, fluorinated chromium oxide which is prepared by fluorinating thermally treated Cr(OH)₃ with hydrogen fluoride, a catalyst prepared by thermally treating a hydrate of CrF₃ in an oxygen-containing atmosphere, and the like.

In the present invention, a part or a whole of the generated gas from the second reaction is used as the diluent gas in the first reactor.

A flow chart of this embodiment is shown in Fig. 1.

In the second reactor, R-133a and hydrogen fluoride are supplied. The reaction product from the second reactor is a gaseous mixture (the "first gaseous mixture") of desired R-134a, unreacted R-133a and hydrogen fluoride, and by-products including R-1122. The first gaseous mixture is directly supplied to the first reactor together with the raw material, namely trichloroethylene.

Trichloroethylene reacts with hydrogen fluoride in the first reactor to form R-133a. Simultaneously, R-1122 reacts with hydrogen fluoride and is reconverted to R-133a. Therefore, the reaction mixture from the first reactor contains R-133a, R-134a, hydrogen fluoride, hydrogen chloride, a small amount of trichloroethylene and some by-products. But, the reaction mixture contains substantially no R-1122.

From the generated gas from the first reactor, hydrogen chloride is removed and then R-134a is separated. Remaining R-133a and hydrogen fluoride are supplied to the second reactor. To the second reactor, a supplemental amount of hydrogen fluoride is added.

In this process, since the heat generated in the first reaction is cooled by the reaction product from the second reaction, the reaction temperature in the first reaction is very easily controlled, and the number of the reactors can be decreased from three to two.

According to the volume of the first reactor, the generated gas from the first reactor contains a small amount of R-1122. In such case, a third reactor which is operated at a temperature of 180 to 300°C is provided after the first reactor. Such third reactor may be a small one.

The reaction in each reactor will be explained.

To the second reactor, R-133a and hydrogen fluoride which is preferably anhydrous are supplied. A molar ratio of HF to R-133a is at least 2. Even when this ratio is smaller than 2, the reaction may proceed but the selectivity decreases unpreferably. The upper limit of this ratio is not limited. As this ratio increases, an amount of recovered and recycled hydrogen fluoride increases so that the production cost increases. In general, the upper limit of this ratio is about 10. To the second reactor, the same catalyst as above may be added.

The reaction temperature is preferably from 300 to 400°C. When the reaction temperature is lower than 300°C, the conversion is very low due to the equilibrium. When it is higher than 400°C, the selectivity is very low.

To the first reactor, a gaseous mixture of trichloroethylene, hydrogen fluoride and R-1122 is supplied. When the exit gas from the second reactor is directly supplied to the first reactor, trichloroethylene is simultaneously supplied in the same mole as that of R-133a which is consumed in the second reactor. Though hydrogen fluoride which reacts with trichloroethylene may be supplied, usually it is not necessary to supply hydrogen fluoride since the gas from the second reactor contains a sufficient amount of hydrogen fluoride.

The first reactor may contain the same catalyst as above.

The reaction temperature may vary with the activity of the catalyst. Usually, as described above, it is from 180 to 300°C.

Each reactor may be any type of a reactor. Since the reactions in the present invention are gas-solid contact reactions, usually, a multi-tubular fixed bed reactor or a fluidized bed reactor can be used. In addition, a moving bed reactor and the like may be used. The types of the first and second reactors may be the same or different.

### PREFERRED EMBODIMENTS OF THE INVENTION

The present invention will be illustrated by following Examples.

### Comparative Example

By heating chromium hydroxide which was precipitated from an aqueous solution of chromium nitride with an aqueous ammonia, a fluorination catalyst was produced. Prior to use, the catalyst was fluorinated with hydrogen fluoride. Forty grams of the catalyst was filled in a Hastelloy C tube of 20 mm in inner diameter and 700 mm in length and heated to 320°C in a nitrogen stream. Then, the supply of nitrogen was stopped and trichloroethylene and hydrogen fluoride were supplied at flow rates of 85 ml/min. and 420 ml/min., respectively. As soon as trichloroethylene and hydrogen fluoride were supplied, an exothermic reaction started and the maximum temperature reached 345°C.

After the produced gas was washed with water and dried, its composition was analyzed by gas chromatography.

The conversion of trichloroethylene was 98 %, and the selectivity was 96 %.

When the reaction was continued under the same conditions, sudden great decrease of the conversion was observed after 400 hours.

### Example 1

Ten grams of the same catalyst as in the Comparative Example were filled in a Hastelloy C tube of 20 mm in inner diameter and 700 mm in length (a second reactor tube) and heated to 360°C in a nitrogen stream. Then, the supply of nitrogen was stopped and R-133a and hydrogen fluoride were supplied at flow rates of 60 ml/min. and 360 ml/min., respectively. The exit gas gas washed with water and dried and its composition was analyzed by gas chromatography. The conversion of R-133a was 30 %, and the selectivities of R-134a and R-1122 were 97 % and 2 %, respectively.

Ten grams of the same catalyst as above was filled in the same Hastelloy C tube (a first reactor tube) and heated to 250°C in a nitrogen stream. Then, the supply of nitrogen was stopped and trichloroethylene was supplied at a flow rate of 18 ml/min. together with the exit gas from the second reactor tube to the first reactor tube. No heat generation was observed. The exit gas from the first reactor tube was analyzed by gas chromatography to find that the conversion of trichloroethylene was 99 % and no R-1122 was detected. The amount of R-134a was not substantially changed.

## Claims

1. A process for preparing 1,1,1,2-tetrafluoroethane comprising the steps of :
(i) fluorinating trichloroethylene in the gas phase with hydrogen fluoride in the presence of a fluorination catalyst in a first reactor to form 1,1,1-trifluoro-2-chloroethane; and
(ii) fluorinating the 1,1,1-trifluoro-2-chloroethane in the gas phase with hydrogen fluoride in the presence of a fluorination catalyst in a second reactor to generate a first gaseous mixture comprising 1,1,1,2-tetrafluoroethane, unreacted 1,1,1-trifluoro-2-chloroethane and 1,1-difluorochloroethylene as a by-product
**characterized in that**:
(a) at least a part of the first gaseous mixture is fed to the first reactor where it acts as a diluent for the fluorination step (i) and where the 1,1-difluorochloroethylene is fluorinated with hydrogen fluoride to 1,1,1-trifluoro-2-chloroethane; and
(b) the 1,1,1,2-tetrafluoroethane is recovered from the mixture of gases resulting from the fluorination step (i).

2. A process according to Claim 1, wherein the fluorination step (i) is conducted at a temperature of 180-300°C.

3. A process according to Claim 1 or Claim 2, wherein the fluorination step (ii) is conducted at a temperature of 300-400°C.

4. A process according to Claim 3, comprising the further step of feeding the mixture of gases resulting from the fluorination step (i) to a third reactor which reduces the amount of 1,1-difluorochloroethylene prior to the step of recovering the 1,1,1,2-tetrafluoroethane from the mixture of gases.

5. A process according to Claim 4, wherein the third reactor is operated at a temperature of 180-300°C.

6. A process according to any preceding Claim wherein the volume of the first gaseous mixture fed to the first reactor is 1-40 times the volume of trichloroethylene.

7. A process according to any preceding Claim wherein all of the first gaseous mixture resulting from the fluorination step (ii) is fed to the first reactor.

8. A process for preparing 1,1,1,2-tetrafluoroethane which comprises the steps of:
(1) reacting trichloroethylene with hydrogen fluoride in the gas phase in the presence of a fluorination catalyst to obtain 1,1,1-trifluoro-2-chloroethane in a first reactor,
(2) reacting 1,1,1-trifluoro-2-chloroethane from the first reactor with hydrogen fluoride in the gas phase in the presence of a fluorination catalyst to obtain 1,1,1,2-tetrafluoroethane and by-product 1,1-difluorochloroethylene in a second reactor,
(3) recycling the entire reaction mixture including 1,1,1,2-tetrafluoroethane and unreacted 1,1,1-trifluoro-2-chloroethane from the second reactor to the first reactor,
(4) reacting 1,1-difluorochloroethylene produced in the second reactor with hydrogen fluoride to reduce the amount of 1,1,-difluorochloroethylene in the first reactor, and,
(5) recovering 1,1,1,2-tetrafluoroethane from the reaction mixture obtained from the first reactor prior to feeding this mixture to the second reactor.

9. A process according to Claim 8, wherein the reaction (1) is carried out at a temperature of 180-300°C, and the reaction (2) is carried out at a temperature of 300-400°C.

10. A process according to Claim 8, wherein the molar ratio of hydrogen fluoride to 1,1,1-trifluoro-2-chloroethane in the second reactor is from 2:1 to 10:1.

11. A process according to Claim 8, wherein the hydrogen fluoride required for both the reactions of steps (1) and (2) is introduced into the second reactor.

12. A process according to Claim 8, wherein the fluorination catalysts used in steps (1) and (2) comprise thermally treated chromium hydroxide.

## Patentansprüche

1. Verfahren zur Herstellung von 1,1,1,2-Tetrafluorethan, das die folgenden Schritte umfasst:
(i) Fluorierung von Trichlorethylen in der Gasphase mit Fluorwasserstoff in Anwesenheit eines Fluorierungskatalysators in einem ersten Reaktor unter Bildung von 1,1,1-Trifluor-2-chlorethan; und
(ii) Fluorierung des 1,1,1-Trifluor-2-chlorethans in der Gasphase mit Fluorwasserstoff Anwesenheit eines Fluorierungskatalysators in einem zweiten Reaktor unter Bildung einer ersten gasförmigen Mischung, die 1,1,1,2-Tetrafluorethan, nicht-reagiertes 1,1,1-Trifluor-2-chlorethan und 1,1-Difluorchlorethylen als Nebenprodukt umfasst, **dadurch gekennzeichnet, dass**
(a) mindestens ein Teil der ersten gasförmigen Mischung in den ersten Reaktor eingebracht wird, wo es als Verdünnungsmittel in dem Fluorierungsschritt (i) fungiert, und worin das 1,1-Difluorchlorethylen mit Fluorwasserstoff zu 1,1,1-Trifluor-2-chlorethan fluoriert wird, und
(b) das 1,1,1,2-Tetrafluorethan aus der Gasmischung, die aus dem Fluorierungsschritt (i) resultiert, abgetrennt wird.

2. Verfahren gemäss Anspruch 1, worin der Fluorierungsschritt (i) bei einer Temperatur von 180-300°C durchgeführt wird.

3. Verfahren gemäss Anspruch 1 oder 2, worin der Fluorierungsschritt (ii) bei einer Temperatur von 300-400°C durchgeführt wird.

4. Verfahren gemäss Anspruch 3, das den weiteren Schritt der Zuführung der aus dem Fluorierungsschritt (i) resultierenden Gasmischung in einen dritten Reaktor umfasst, worin die Menge an 1,1-Difluorchlorethylen vor dem Schritt der Abtrennung von 1,1,1,2-Tetrafluorethan aus der Gasmischung verringert wird.

5. Verfahren gemäss Anspruch 4, worin der dritte Reaktor bei einer Temperatur von 180-300°C betrieben wird.

6. Verfahren gemäss mindestens einem der vorangegangenen Ansprüche, worin das Volumen der ersten gasförmigen Mischung, die in den ersten Reaktor eingebracht wird, das 1- bis 40-fache des Volumens an Trichlorethylen beträgt.

7. Verfahren gemäss mindestens einem der vorangegangenen Ansprüche, worin die gesamte Gasmischung, die aus dem Fluorierungsschritt (ii) resultiert, in den ersten Reaktor eingebracht wird.

8. Verfahren zur Herstellung von 1,1,1,2-Tetrafluorethan, das folgende Schritt umfasst:
(1) Reaktion von Trichlorethylen mit Fluorwasserstoff in der Gasphase in Gegenwart eines Fluorierungskatalysators unter Erhalt von 1,1,1-Trifluorchlorethan in einem ersten Reaktor;
(2) Reaktion von 1,1,1-Trifluor-2-chlorethan aus dem ersten Reaktor mit Fluorwasserstoff in der Gasphase in Gegenwart eines Fluorierungskatalysators unter Erhalt von 1,1,1,2-Tetrafluorethan und dem Nebenprodukt 1,1-Difluorchlorethylen in einem zweiten Reaktor;
(3) Recyclierung der gesamten Reaktionsmischung, einschliesslich 1,1,1,2-Tetrafluorethan und nicht-reagiertem 1,1,1-Trifluor-2-chlorethan aus dem zweiten Reaktor in den ersten Reaktor;
(4) Reaktion von 1,1-Difluorchlorethylen, das in dem zweiten Reaktor erzeugt wurde, mit Fluorwasserstoff unter Reduzierung der Menge an 1,1-Difluorchlorethylen in dem ersten Reaktor; und
(5) Abtrennung von 1,1,1,2-Tetrafluorethan aus der in dem ersten Reaktor erhaltenen Gasmischung, bevor diese Mischung in den zweiten Reaktor eingeführt wird.

9. Verfahren gemäss Anspruch 8, worin die Reaktion (1) bei einer Temperatur von 180-300°C und die Reaktion (2) bei einer Temperatur von 300-400°C durchgeführt wird.

10. Verfahren gemäss Anspruch 8, worin das Molverhältnis von Fluorwasserstoff zu 1,1,1-Trifluor-2-chlorethan in dem zweiten Reaktor im Bereich von 2:1 bis 10:1 liegt.

11. Verfahren gemäss Anspruch 8, worin der Fluorwasserstoff, der für beide Reaktionen in den Schritten (1) und (2) benötigt wird, in den zweiten Reaktor eingebracht wird.

12. Verfahren gemäss Anspruch 8, worin die in den Schritten (1) und (2) verwendeten Fluorierungskatalysatoren thermisch behandeltes Chromhydroxid umfassen.

## Revendications

1. Procédé de préparation du 1,1,1,2-tétrafluoroéthane qui comprend les étapes consistant à :
(i) fluorer le trichloroéthylène en phase gazeuse avec du fluorure d'hydrogène en présence d'un catalyseur de fluoration dans un premier réacteur pour former du 1,1,1-trifluoro-2-chloroéthane ; et
(ii) fluorer le 1,1,1-trifluoro-2-chloroéthane en phase gazeuse avec du fluorure d'hydrogène en présence d'un catalyseur de fluoration dans un second réacteur pour engendrer un premier mélange gazeux contenant du 1,1,1,2-tétrafluoroéthane, du 1,1,1-trifluoro-2-chloroéthane non entré en réaction et du 1,1-difluorochloroéthylène comme sous-produit, **caractérisé en ce que** : (a) au moins une partie du premier mélange gazeux est introduit dans le premier réacteur où il sert de diluant pour l'étape de fluoration (i) et où le 1,1-difluorochloroéthylène est fluoré avec le fluorure d'hydrogène en 1,1,1-trifluoro-2-chloroéthane; et (b) le 1,1,1,2-tétrafluoroéthane est récupéré du mélange de gaz résultant de l'étape de fluoration (i).

2. Procédé selon la revendication 1, où l'étape de fluoration (i) s'effectue à une température de 180 à 300°C.

3. Procédé selon la revendication 1 ou la revendication 2, où l'étape de fluoration (ii) est effectuée à une température de 300 à 400°C.

4. Procédé selon la revendication 3, comprenant l'étape supplémentaire consistant à introduire le mélange de gaz provenant de l'étape de fluoration (i) dans un troisième réacteur qui réduit la quantité de 1,1-difluoroéthylène avant l'étape de récupération du 1,1,1,2-tétrafluoroéthane du mélange de gaz.

5. Procédé selon la revendication 4, où le troisième réacteur fonctionne à une température de 180 à 300°C.

6. Procédé selon l'une quelconque des revendications précédentes, où le volume du premier mélange gazeux introduit dans le premier réacteur représente de 1 à 40 fois le volume du trichloroéthylène.

7. Procédé selon l'une quelconque des revendications précédentes, où la totalité du premier mélange gazeux provenant de l'étape de fluoration (ii) est introduite dans le premier réacteur.

8. Procédé de préparation du 1,1,1,2-tétrafluoroéthane, qui comprend les étapes consistant à :
(1) faire réagir du trichloroéthylène avec du fluorure d'hydrogène en phase gazeuse en présence d'un catalyseur de fluoration pour obtenir du 1,1,1-trifluoro-2-chloroéthane dans un premier réacteur,
(2) faire réagir le 1,1,1-trifluoro-2-chloroéthane du premier réacteur avec du fluorure d'hydrogène en phase gazeuse en présence d'un catalyseur de fluoration pour obtenir du 1,1,1,2-tétrafluoroéthane et du 1,1-difluorochloroéthylène formé à titre de sous-produit dans un second réacteur,
(3) recycler le mélange de réaction entier comprenant le 1,1,1,2-tétrafluoroéthane et le 1,1,1-trifluoro-2-chloroéthane non entré en réaction depuis le second réacteur vers le premier réacteur,
(4) faire réagir le 1,1-difluorochloroéthylène produit dans le second réacteur avec du fluorure d'hydrogène pour réduire la quantité de 1,1-difluorochloroéthylène dans le premier réacteur et
(5) récupérer le 1,1,1,2-tétrafluoroéthane du mélange de réaction obtenu à partir du premier réacteur avant d'introduire ce mélange dans le second réacteur.

9. Procédé selon la revendication 8, où la réaction (1) est conduite à une température de 180-300°C et la réaction (2) est conduite à une température de 300-400°C.

10. Procédé selon la revendication 8, où le rapport molaire du fluorure d'hydrogène au 1,1,1-trifluoro-2-chloroéthane dans le second réacteur varie de 2:1 à 10:1.

11. Procédé selon la revendication 8, où le fluorure d'hydrogène nécessaire aux deux réactions des étapes (1) et (2) est introduit dans le second réacteur.

12. Procédé selon la revendication 8, où les catalyseurs de fluoration utilisés dans les étapes (1) et (2) comprennent de l'hydroxyde de chrome soumis à un traitement thermique.
